# EUROPEAN PATENT SPECIFICATION

(11) **EP 1 273 808 B1**
(45) Date of publication and mention of the grant of the patent: **05.04.2006**
(21) Application number: 01123923.3
(22) Date of filing: 06.10.2001
(51) Int. Cl.: F15C 5/00

(54) **Adjustable micro valve and method of setting and actuating it**
Einstellbares Mikroventil und Verfahren zur Einstellung und Betätigung
Micro-soupape réglable et procédé de réglage et manoeuvre

(30) Priority: 02.07.2001 EP 01116013
(43) Date of publication of application: 08.01.2003
(73) Proprietor: Medos International S.a.r.l., 2400 Le Locle (CH)
(72) Inventor: Ginggen, Alec, 3225 Müntschemier (CH)
(74) Representative: Micheli & Cie

(56) References cited:
- EP-A- 0 778 043
- WO-A-99/38551
- WO-A-99/39118
- DE-C- 19 645 725
- US-A- 5 619 177
- US-A- 5 697 951
- US-A- 6 149 123

## Description

The present invention relates to a method of setting and/or actuating a multi-stable valve used in fluidic or in micro fluidic applications. Another object of the invention is an adjustable multi stable valve for use in medical devices implanted in a human body.

More specifically, the invention relates to a micro valve having at least two stable states at operating temperature. An opening pressure and a resistance to fluid flow correspond to each state of the valve. The valve may be actuated non-invasively, by telemetry for example, thanks to an external device, providing an adjustable opening pressure valve or alternatively a valve assembly with adjustable resistance to flow.

The valve object of the present invention has a wide range of applications in different fields (medical, hydraulics, micro-engineering, ...). For example, in the medical field related to the treatment of hydrocephalic patients, it is necessary to install a shunt system that derives the excess of liquid from the brain to the peritonea or to another cavity of the patient. Some existing shunt systems comprise an adjustable valve that allows the surgeon to modify non-invasively the valve opening pressure after implantation. These existing implantable valves for the treatment of hydrocephalic patients have successfully shown that the feature allowing the surgeon to adjust non-invasively the valve opening pressure after implantation is extremely useful. Nevertheless, there are some drawbacks associated with devices of this type that can be summarised as follows:

These known implants do not provide the user with any feedback during or after adjustment of the valve opening pressure. Therefore, it might be necessary to take an X-ray for checking the valve setting. Furthermore, the valve can be misadjusted by strong magnetic fields, such as those generated by a permanent magnet found for example in magnetic resonance imaging devices.

Finally the existing valves are sometimes blocked due to an accumulation of bio-substance on the mechanical parts of the valve mechanism.

Some other known electromechanical or pneumatic valves require energy for remaining in one or more working positions and are not suitable for human or animal implantation due to their size and / or their lack of tightness.

WO 99/39118 and US 5 619 177 describe valves which are made of a shape memory alloy (SMA) material and which take two different, permanent shapes when cold or heated to two different predetermined temperatures.

The valve object of the present invention overcomes the problems exposed above by providing a micro valve having at least two stable states at operating temperature. The valve according to the invention does not require energy at rest during normal operation and is insensitive to magnetic fields by design. Since the valve setting may be adjusted without mechanical movement of any parts, the valve is less sensitive to blockage due to an accumulation of bio substances.

The actuation concept is based on temperature changes above and below body temperature. Energy is only required to change the valve from one state to the other. Valves for the treatment of hydrocephalic patients, as well as valves for all kind of implantable pumps constitute major applications of that concept that may be extended to other fields.

A method for setting an implantable valve as defined in claim 1 as well as a micro valve having the characteristics recited in claim 7 obviate the above mentioned drawbacks.

Further features and other objects and advantages of this invention will become clear from the following detailed description made with reference to the accompanying drawings illustrating in a schematic and non-limiting way three embodiments of a multi stable micro valve according to the invention and in which:
Figure 1 is a graph showing the typical temperature hysteresis of shape memory alloy (SMA).
Figure 2 is a graph showing the typical stress-strain characteristics of a shape memory alloy in each of its states.
Figure 3 is a schematic perspective top view of a first embodiment of a micro valve according to the invention.
Figure 4 is a bottom perspective view according to the first embodiment shown at figure 3.
Figure 5 is cross sectional view of the first embodiment of the valve shown at figure 3.
Figure 6 is a perspective top view of a second embodiment of a micro valve according to the invention.
Figure 7 is perspective bottom view of the second embodiment depicted in figure 6.
Figure 8 is schematic cross sectional view of the second embodiment depicted in figure 6.
Figure 9 is schematic perspective top view of a third embodiment of a micro valve according to the invention.
Figure 10 is a perspective bottom view of the third embodiment depicted in figure 9.
Figure 11 is a perspective top view of an implantable assembly incorporating a valve according to the invention, the top cover being exploded.
Figure 12 is a perspective view of the assembly depicted in figure 11 with the bottom cover exploded.
Figure 13 is a bottom perspective view of the assembly shown at figures 11 and 12.
Figure 14 is an exploded perspective view of an implantable pump embodying a valve according to the invention.

In the following disclosure, reference will be made to shape memory alloys, hereafter called SMA material. The properties and characteristics of such materials are briefly described in the following.

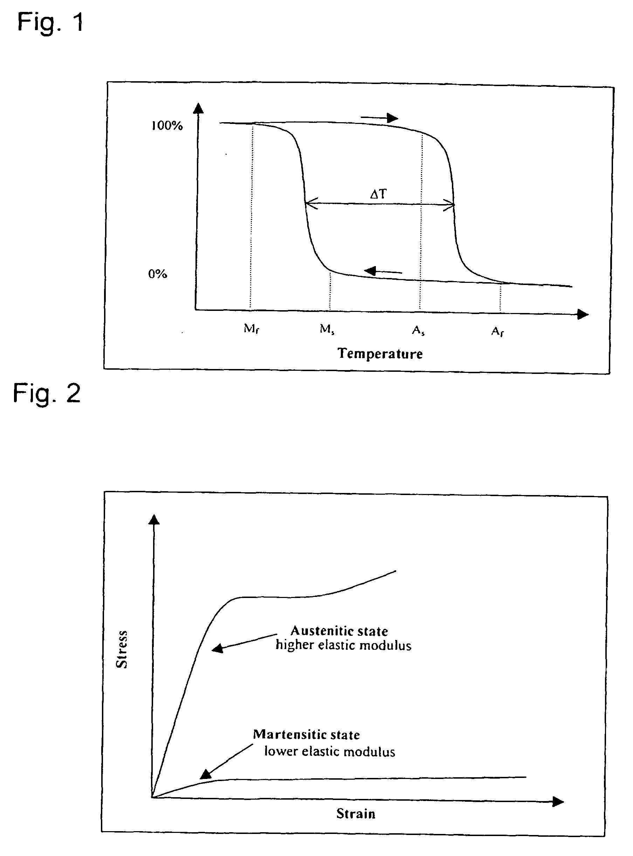

SMA material is characterised by reversible metallurgical phase transformations that are activated either by temperature change or by induced stress. Below a range of transition temperature, the material is in the martensitic state, whereas above that temperature range, the material is in the austenitic state. The transformation occurs across a range of temperatures which are commonly named Aₛ (start) and A_{f} (finish) for the transformation from martensitic to austenitic state and Mₛ (start) and M_{f} (finish) for the transformation from austenitic to martensitic state as referenced in figure 1. These transformations are reversible so that the material may be treated to assume different shapes in each of the two phases, and can reversibly switch between the two shapes when transformed from one state to the other. More commonly, the material is treated to only return to a shape upon transformation to the austenitic phase a biasing force, acting against the SMA material returns it to its alternate shape upon transformation to the martensitic phase.

Most of the temperature cycles of the SMA materials have a hysteresis ΔT, as illustrated on the graph of figure 1.

The elastic modulus of the SMA material depends on its metallurgical state. Figure 2 shows a typical stress-strain graph of a SMA material in both states. It appears clearly that the austenitic state has a higher elastic modulus than the martensitic state. Upon initial loading, the stress-strain curve is roughly linear and the Young's modulus corresponds to the slope of the curve in the initial loading region. For materials tested at temperatures just above the A_{f} temperature, if the material is further deformed beyond this initial loading region, it will experience a stress-induced martensitic transformation. The point on the stress-strain curve at which the stress-induced martensitic transformation begins can be called the Mₛ^{σ}.

In the martensitic state, the elastic modulus is lower than in the austenitic state, and the corresponding Mₛ^{σ} (in this case, the stress required to rearrange the pre-existing martensitic phase) is also lower.

The invention makes use of the change in mechanical properties (mainly Young's modulus) of an array of actuators in SMA material when a transition between the two metallurgical states occurs.

For medical implantable devices, the SMA material is preferably chosen within SMA materials having a working temperature corresponding to body temperature located between Mₛ and Aₛ. In that case, the material is stable in both states at rest. Heating the material above A_{f} will transform it into austenite (higher modulus material). Cooling the material below M_{f} will transform it into martensite (lower modulus material). While the effect is most pronounced with the temperature of use located between Mₛ and Aₛ, the effect can be observed to some extent at a number of temperatures in the broader range between M_{f} and A_{f}.

For example TiNi (Nitinol) is a good choice for the actuating members of a valve according to the invention as it is biocompatible. Further, TiNi can be manufactured such that body temperature is located between Mₛ and Aₛ. A TiNi material manufactured to meet this criterion might have the following characteristics: Martensitic transformation: M_{f}= 24°C, Mₛ= 36°C, Austenitic transformation: Aₛ = 54°C, A_{f}=71°C, with a hysteresis : ΔT ~ 35°C. Note that the transformation temperatures for a particular material also change with stress, so that the temperatures of the starting material must be selected to appropriately accommodate the variability due to the operating stresses of the particular application.

Fine tuning the temperature cycle and the mechanical properties may be achieved by playing with the chemical composition and thermomechanical processing of the material.

As it will be described in detail with reference to the figures, the micro valve object of the invention comprises an array of actuators or actuating members made of a SMA material that interact either directly with the fluid path or with an elastic mean, the tension of which being modified by said array of SMA actuators.

The SMA material is selected to have two stable metallurgical states at the temperature of use, e.g., body temperature. The metallurgical state can be changed either by cooling or by heating the SMA actuator. One of the metallurgical states has a higher elastic-modulus, whereas the other state has a lower elastic modulus.

The heating is obtained by circulating a current through or in the proximity of the SMA material (Joule effect). The cooling is achieved thanks to a Peltier cell or an array of Peltier cells integrated in the base plate of the valve, in the vicinity of the SMA actuators.

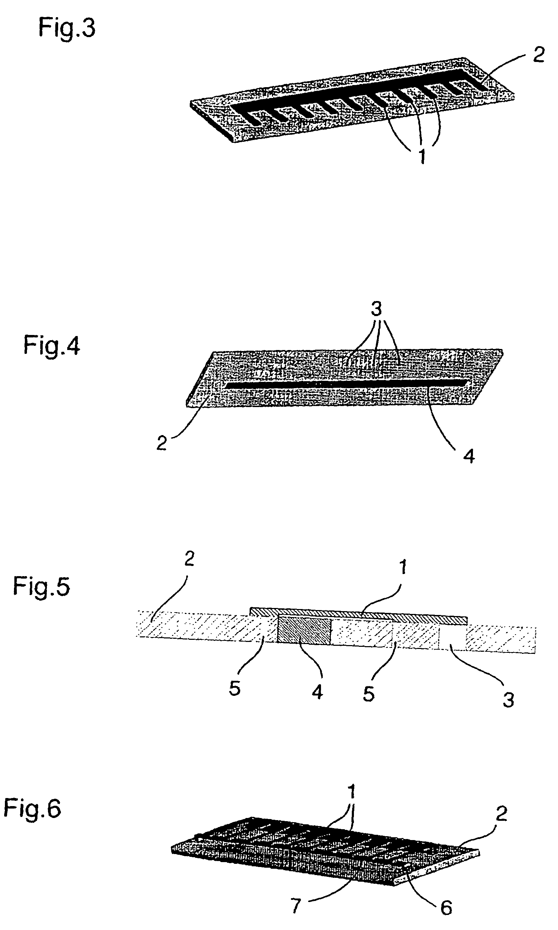

With reference to figure 3,4 and 5, a first embodiment of a micro valve with adjustable opening pressure is illustrated.

The fluid path crosses a base plate 2 having an array of orifices 3, closed by the free extremity of a corresponding array of actuating members 1. The base plate 2 is preferably made of a glass type material like Pyrex for example. The geometry of the orifices 3 is identical across the array, which ensures that the resistance to fluid is the same for each single orifice 3. The array of actuating members 1 comprises, in this embodiment, an elongated body from which extend perpendicularly elongated actuating members. Some other configurations are of course possible. The actuating members 1 are made of SMA material, preferably TiNi, and their geometry is chosen so that each fluid path 3 can be considered as closed when the corresponding actuating member 1 is in the austenitic state and open in the martensitic state.

A Peltier cell 4 is integrated in the base plate 2, and allows, once energised, the cooling of the array of actuating members 1.

Each actuating member 1 may be heated individually by circulating an electrical current through the connectors 5 bounded to each of the actuating members 1.

The valve setting is modified in the following manner. First, the temperature of the array of SMA actuating members 1 is decreased to a temperature substantially lower than Mₛ (preferably below M_{f}) by energising the Peltier cell 4. This transforms all or part of the actuating members 1 to the martensitic state (lower modulus). Then, at least one actuating member is selected within the array and the temperature of all actuating members 1 except the previously selected is increased to a temperature substantially higher than Aₛ (preferably above A_{f}) This is achieved by circulating an electrical current through the connectors 5 connected to the actuating members 1. Once the higher temperature is reached, all or part of the actuating members 1 are in the austenitic state (higher modulus) except the selected actuating member which remains all or partially in the martensitic state thus determining the opening pressure of the valve.

As an alternative, the array of SMA actuators may be first heated to a temperature at which an austenitic transformation occurs and then at least one selected actuating member is cooled down to a temperature at which a martensitic transformation occurs. For implementing this alternate method, an array of Peltier cells is provided. Each Peltier cell forming the array being located in the vicinity of an actuating member so as to enable the individual cooling of each actuating member.

The size and geometry of each actuating member 1 forming the array can be adjusted for providing different opening pressure depending on which actuating member remains in the martensitic state.

Figures 6, 7 and 8 depict another embodiment of a valve with an adjustable opening pressure. The base plate 2 has only one orifice 3 through which the fluid may flow. A ball 6 is maintained in the seat of the orifice 3 thanks to an elastic element like a flexible flat spring 7 for example. The spring 7 need not be made of a SMA material.

An array of SMA actuating members 1 is arranged perpendicularly to the spring 7 and the free end of each actuating member 1 interacts with the spring 7. Depending on the metallurgical state of the SMA actuating members 1, the length of the spring allowed to move freely is restricted. The force applied to the ball is determined by the tension of the spring 7 which varies with the metallurgical states of the actuating members 1.

A Peltier cell is integrated in the base plate 2 in the vicinity of the SMA array of actuating members 1. Upon activation, the Peltier cell cools the array and all the actuating members 1 change to martensitic state. Each of the actuating members 1 may then be individually heated to a temperature at which an austenitic transformation occurs. This determines the length of activation of the spring 7 and therefore the opening pressure of the valve.

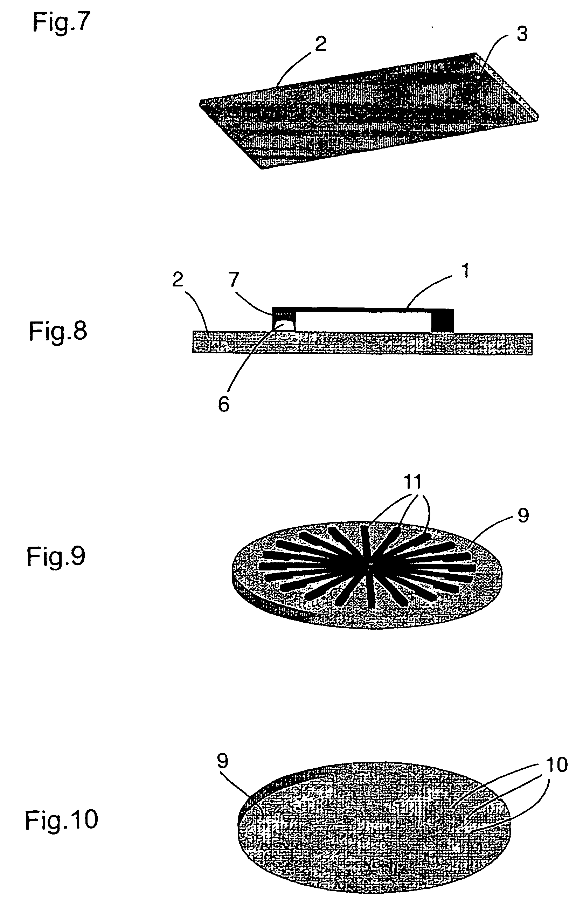

With reference to figures 9 and 10, a third embodiment of a valve according to the invention is disclosed. This embodiment provides a valve with an adjustable resistance to flow. A circular base plate 9 comprises, on its periphery, an array of openings 10 through which a fluid may flow. An array of actuating members 11 is arranged on the base plate 9 so that the free end of each actuating member 11 closes a corresponding opening 10 of the base plate. The SMA actuating members 11 are preferably extending from the centre of the base plate 9 to the periphery of said plate.

In this embodiment, all the actuating members 11 have the same geometry but the geometry of the orifices 10 may differ in order to provide a range of different resistances to flow. A Peltier cell or an array of Peltier cells is integrated in the base plate 9, preferably in the centre of the base plate so as to enable cooling of the complete array of SMA actuating members 11.

The setting or the actuating of the valve is similar to what has been disclosed in reference to the first embodiment at figure 3 to 5.

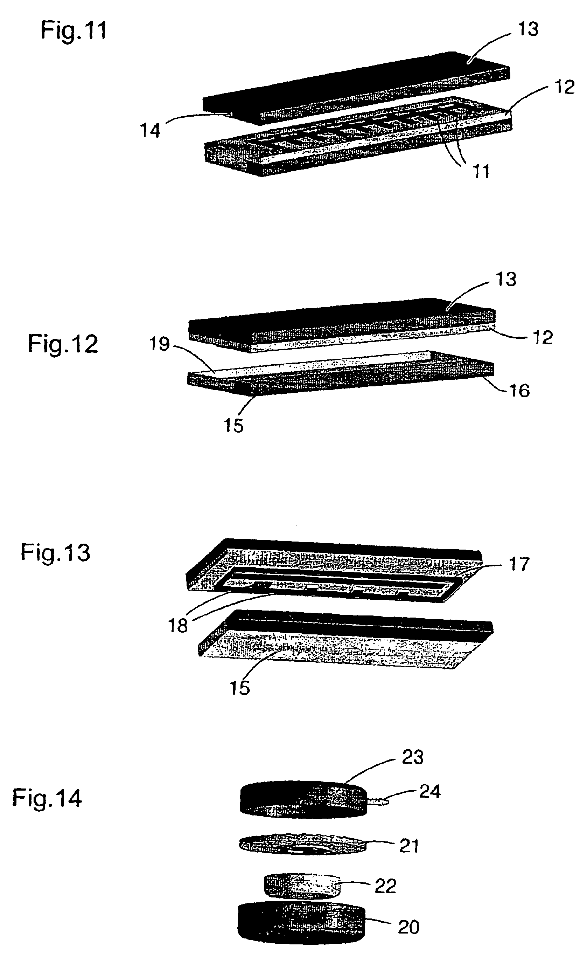

Figure 11, 12 and 13 illustrate an implantable valve with adjustable opening pressure. The implantable valve comprises a valve assembly 12 according to one of the first or second embodiment disclosed above. A top cover 13 having a fluid outlet 14 is adapted to receive the valve assembly 12. An antenna 17 as well as the necessary electronic components 18 to power and control the valve assembly by telemetry are integrated on the bottom of the base plate of the valve assembly 12. A bottom cover 15 having a fluid inlet 16 and a leak tight compartment 19 for protecting the electronic components closes the structure.

The user may then power the assembly by telemetry and select non-invasively the opening pressure from outside the body by firstly cooling the SMA array of actuating members 11 and then selectively heating by Joule effect one or more actuating members 11. The electronic components 18 integrate a feedback mechanism that can be used to confirm that the correct actuating member 11 or array of actuating members have been heated.

A valve according to the invention may also be used in an implantable drug delivery pump. A few existing adjustable implantable pumps allow the user (patient and/or doctor) to select non-invasively a flow rate of chemicals to inject, thanks to an external programming unit. The existing devices can be divided in two main categories: the active or passive pumping mechanisms. In the first case, a battery energises a pump that regulates the flow rate of chemicals. In the second case, a pressure reservoir "pushes" the chemicals out of the pump. The later concept is very elegant since the pumping does not require energy. Nevertheless, the regulation of the fluid flow is ensured by a valve, the opening of which depends on the power delivered to the valve. Therefore, a battery is still required.

Thanks to a valve according to the invention, when used in an implantable adjustable pump, the energy consumption problem is solved, since energy is only required to change the flow setting of the pump.

In the current products, energy is required continuously to keep the valve open. An adjustable passive pressurised pump embodying a valve according to the invention will now be disclosed with reference to figure 14.

The implantable pump comprises a pressurised reservoir 20 that contains the drug substance to administrate. A valve assembly 21 as described with reference to the third embodiment shown in figures 9 and 10 constitutes the adjustable flow resistance valve of the pump. The bottom of the base plate of the valve assembly 21 incorporates electronic components and an antenna that are used to power and control the valve non invasively by telemetry. A leak tight cover 22 protects the bottom face of the base plate and the electronics components, avoiding contact with the pressurised liquid contained in the reservoir 20. A top cover 23 having a fluid outlet 24 closes the structure.

The user may select the resistance of the valve from outside with a dedicated reading unit and therefore regulates the outflow of chemicals contained in the pressurised reservoir.

Many advantages are achieved with a valve according to the invention. Firstly, as the valve has multi stable states, energy is only required to switch from one state to the other. No energy is needed to maintain a selected state. Each state can either correspond to a selected opening pressure or to a flow resistance, depending on the application.

Second, the valve settings can be adjusted without a movement of any part. Only the elastic modulus of the material is modified and therefore the valve is less sensitive to blockage by clogs and other bio-substances.

The energy required is the energy needed to power a Peltier cell, and the energy for heating the actuating members. This energy can be provided to the implantable device by telemetry avoiding the use of batteries.

For medical applications, and more particularly for implantable adjustable valves or pumps as previously described, the choice of the SMA material is of importance. It must be chosen from the SMA materials that have two stable states at a temperature in the vicinity of the body temperature. Furthermore, the SMA material ideally should fulfill the following conditions. Mₛ < T < Aₛ where T is the temperature of the human body and an hysteresis ΔT, comprised between 10 and 40 degrees centigrade. TiNi (Nitinol) is a material that fulfills these requirements and which is also biocompatible.

It will be appreciated by persons skilled in the art that the present invention is not limited by what has been disclosed above, particularly with regards to the field of use of the valve which may be integrated in other fluidic devices.
Furthermore, the present invention may include combinations and subcombinations of the various features disclosed as well as modifications and extensions thereof which fall under the scope of the following claims.

## Claims

1. A method of setting an adjustable micro valve comprising an array of actuating members (1) made of an SMA material, **characterised in that** the SMA material presents an hysteresis cycle between a first and a second temperature (Mf, Af) and is selected so that an ambient temperature of use of said valve falls within said hysteresis cycle, the SMA material thus having a first and a second stable state at said ambient temperature of use and having a lower elastic modulus when in the first state compared to when in the second state, the method comprising the step of switching at least one actuating member (1) from one state to another by temperature changes.

2. A method according to claim 1, **characterised in that** said switching step comprises the following steps:
- cooling the array of actuating members (1) from the ambient temperature of use to a temperature equal to or below a temperature (Mₛ; M_{f}) at which a transformation from the second state to the first state occurs so that the entire array of actuating members (1) is either fully or partially in the first state,
- selecting at least one of the actuating members (1) corresponding to a pre-determined opening pressure or resistance to flow,
- heating individually each of the actuating members (1) except the previously selected member to a temperature equal to or above a temperature (Aₛ; A_{f}) at which a transformation from the first state to the second state occurs.

3. A method according to claim 1, **characterised in that** said switching step comprises the following steps:
- heating the array of actuating members (1) from the ambient temperature of use to a temperature equal to or above a temperature (Aₛ; A_{f}) at which a transformation from the first state to the second state occurs so that the entire array of actuating members (1) is either fully or partially in the second state,
- selecting at least one of the actuating members (1) corresponding to a pre-determined opening pressure or resistance to flow,
- cooling individually the selected actuating members to a temperature equal to or below a temperature (Mₛ; M_{f}) at which a transformation from the second state to the first state occurs.

4. A method according to any one of claims 1 to 3, **characterised in that** said ambient temperature of use is the body temperature.

5. A method according to claim 4, **characterised in that** the SMA material has an hysteresis comprised between 10 and 40 degrees centigrade.

6. A method according to any one of claims 1 to 5, **characterised in that** the SMA material has the same shape in the first and second states.

7. An adjustable micro valve comprising a base element (2) with at least one passage (3) for the fluid flow, at least one array of actuating members (1) made of an SMA material arranged on one face of the base element (2), means (4) for cooling the actuating members and means (5) for heating the actuating members, **characterised in that** the SMA material presents an hysteresis cycle between a first and a second temperature (Mf, Af) and is selected so that an ambient temperature of use of said valve falls within said hysteresis cycle, the SMA material thus having a first and a second stable state at said ambient temperature of use and having a lower elastic modulus when in the first state compared to when in the second state.

8. An adjustable micro valve according to claim 7, **characterised in that** the SMA material has the same shape in the first and second states.

9. An adjustable micro valve according to claim 7 or 8, **characterised in that** it further comprises an elastic element (7) securing a ball (6) in the seat of an opening (3) and **in that** the actuating members (1) are conformed so that their respective free ends interact with the elastic element (7) allowing alteration of its elasticity.

10. An adjustable micro valve according to claim 7 or 8, **characterised in that** the actuating members (1; 11) are conformed so that their respective free ends close corresponding orifices (3; 10) in the base element (2; 9).

11. An adjustable micro valve according to any one of claims 7 to 10, **characterised in that** the cooling means consist of at least one Peltier cell (4) integrated in the base element (2) in the vicinity of the array of SMA actuating members (1).

12. An adjustable micro valve according to any one of claims 7 to 11 **characterised in that** the actuating members (1) are made of TiNi.

13. An implantable micro valve having an adjustable opening pressure **characterised in that** it comprises an upper cover (13) having a fluid outlet (14) and a bottom cover (15) having a fluid inlet (16) embodying a micro valve (12) according to any one of claims 7 to 12 and **in that** it further comprises electronic means (18) and an antenna (17) for energising and controlling the actuation of the valve (12) by telemetry thanks to an external unit.

14. An implantable pump **characterised in that** it comprises an upper cover (23) having a fluid outlet (24), a pressurised reservoir (20), a valve assembly (21) according to claim 7 and **in that** it further comprises electronic means and an antenna for energising and controlling the actuation of the pump by telemetry thanks to an external unit.

## Revendications

1. Procédé de mise en place d'une micro-valve ajustable comportant un réseau d'éléments d'actionnement (1) réalisé en un matériau d'alliage à mémoire de forme (AMF), **caractérisé en ce que** le matériau AMF présente un cycle d'hystérésis entre des première et seconde températures (Mf, Af), et est sélectionné de sorte qu'une température ambiante d'utilisation de ladite valve tombe durant ledit cycle d'hystérésis, le matériau AMF ayant ainsi des premier et second états stables dans ladite température ambiante d'utilisation, et ayant un module d'élasticité inférieur dans le premier état par comparaison au second état, le procédé comportant l'étape consistant à commuter au moins un élément d'actionnement (1) à partir d'un premier état vers l'autre par des changements de température.

2. Procédé selon la revendication 1, **caractérisé en ce que** ladite étape de commutation comporte les étapes suivantes :
- refroidir le réseau d'éléments d'actionnement (1) à partir de la température ambiante d'utilisation vers une température égale ou inférieure à une température (Mₛ; M_{f}) à laquelle une transformation à partir du second état vers le premier état survient de sorte que tout le réseau d'éléments d'actionnement (1) est complètement ou partiellement dans le premier état,
- sélectionner au moins un des éléments d'actionnement (1) correspondant à une pression d'ouverture ou une résistance à l'écoulement prédéterminée,
- chauffer individuellement chacun des éléments d'actionnement (1) excepté l'élément sélectionné précédemment jusqu'à une température égale ou supérieure à une température (Aₛ ; A_{f}) à laquelle une transformation du premier état vers le second état survient.

3. Procédé selon la revendication 1, **caractérisé en ce que** ladite étape de commutation comporte les étapes suivantes :
- chauffer le réseau d'éléments d'actionnement (1) à partir de la température ambiante d'utilisation jusqu'à une température égale ou supérieure à une température (Aₛ ; A_{f}) à laquelle une transformation à partir du premier état vers le second état survient, de sorte que tout le réseau d'éléments d'actionnement (1) est complètement ou partiellement dans le second état,
- sélectionner au moins un des éléments d'actionnement (1) correspondant à une pression d'ouverture ou une résistance à l'écoulement prédéterminée,
- refroidir individuellement les éléments d'actionnement sélectionnés jusqu'à une température égale ou inférieure à une température (Mₛ ; M_{f}) à laquelle une transformation à partir du second état vers le premier état survient.

4. Procédé selon l'une quelconque des revendications 1 à 3, **caractérisé en ce que** ladite température ambiante d'utilisation est la température du corps.

5. Procédé selon la revendication 4, **caractérisé en ce que** le matériau AMF a une hystérésis comprise entre 10 et 40 degrés centigrade.

6. Procédé selon l'une quelconque des revendications 1 à 5, **caractérisé en ce que** le matériau AMF a la même forme dans les premier et second états.

7. Micro-valve ajustable comportant un élément de base (2) ayant au moins un passage (3) pour l'écoulement de fluide, au moins un réseau d'éléments d'actionnement (1) réalisés en un matériau AMF agencé sur une première face de l'élément de base (2), des moyens (4) pour refroidir les éléments d'actionnement et des moyens (5) pour chauffer les éléments d'actionnement, **caractérisée en ce que** le matériau AMF présente un cycle d'hystérésis entre des première et seconde températures (Mf ; Af), et est sélectionné de sorte qu'une température ambiante d'utilisation de ladite valve tombe durant ledit cycle d'hystérésis, le matériau AMF ayant ainsi des premier et second états stables dans ladite température ambiante d'utilisation, et ayant un module d'élasticité inférieur dans le premier état par comparaison au second état.

8. Micro-valve ajustable selon la revendication 7, **caractérisée en ce que** le matériau AMF a la même forme dans les premier et second états.

9. Micro-valve ajustable selon la revendication 7 ou 8, **caractérisée en ce qu'**elle comporte en outre un élément élastique (7) fixant une bille (6) dans le siège d'une ouverture (3), et **en ce que** les éléments d'actionnement (1) sont conformés de sorte que leurs extrémités libres respectives interagissent avec l'élément élastique (7), en permettant une modification de son élasticité.

10. Micro-valve ajustable selon la revendication 7 ou 8, **caractérisée en ce que** les éléments d'actionnement (1 ; 11) sont conformés de sorte que leurs extrémités libres respectives ferment des orifices correspondant (3 ; 10) dans l'élément de base (2 ; 9).

11. Micro-valve ajustable selon l'une quelconque des revendications 7 à 10, **caractérisée en ce que** les moyens de refroidissement sont constitués d'au moins une cellule de Peltier (4) intégrée dans l'élément de base (2) à proximité du réseau d'éléments d'actionnement AMF (1).

12. Micro-valve ajustable selon l'une quelconque des revendications 7 à 11, **caractérisée en ce que** les éléments d'actionnement (1) sont réalisés en TiNi.

13. Micro-valve implantable ayant une pression d'ouverture ajustable, **caractérisée en ce qu'**elle comporte un recouvrement supérieur (13) ayant une sortie de fluide (14), et un recouvrement inférieur (15) ayant une entrée de fluide (16) constituant un mode de réalisation d'une micro-valve (12) selon l'une quelconque des revendications 7 à 12, et **en ce qu'**elle comporte en outre des moyens électroniques (18) et une antenne (17) pour mettre sous tension et commander l'actionnement de la valve (12) par télémétrie grâce à une unité externe.

14. Pompe implantable, **caractérisée en ce qu'**elle comporte un recouvrement supérieur (23) ayant une sortie de fluide (24), un réservoir mis sous pression (20), un ensemble formant valve (21) selon la revendication 7, et **en ce qu'**elle comporte en outre des moyens électroniques et une antenne pour mettre sous tension et commander l'actionnement de la pompe par télémétrie grâce à une unité externe.

## Patentansprüche

1. Verfahren zum Einstellen eines einstellbaren Mikroventils mit einer Anordnung von aus einem Formgedächtnislegierungs- (FGL-) Material gefertigten Stellgliedern (1), **dadurch gekennzeichnet, dass** das FGL-Material eine Hystereseschleife zwischen einer ersten und einer zweiten Temperatur (Mf, Af) aufweist und so ausgewählt ist, dass eine Umgebungstemperatur für die Benutzung des Ventils in diese Hystereseschleife fällt, so dass das FGL-Material bei dieser Umgebungstemperatur für die Benutzung einen ersten und einen zweiten stabilen Zustand sowie einen Elastizitätsmodul besitzt, der im ersten Zustand niedriger als im zweiten Zustand ist, wobei das Verfahren den Schritt umfasst, zumindest ein Stellglied (1) durch Temperaturveränderungen von dem einen in den anderen Zustand zu schalten.

2. Verfahren nach Anspruch 1, **dadurch gekennzeichnet, dass** der Schaltschritt die folgenden Schritte umfasst:
- Abkühlen der Anordnung von Stellgliedern (1) von der Umgebungstemperatur für die Benutzung auf eine Temperatur bei oder unterhalb einer Temperatur (Mₛ; M_{f}), bei der eine Umwandlung vom zweiten in den ersten Zustand auftritt, so dass sich die gesamte Anordnung von Stellgliedern (1) entweder ganz oder teilweise im ersten Zustand befindet,
- Auswählen zumindest eines der Stellglieder (1), das einem vorbestimmten Öffnungsdruck oder Strömungswiderstand entspricht,
- individuelles Erhitzen jedes der Stellglieder (1) ausser dem im Voraus ausgewählten Glied, auf eine Temperatur bei oder oberhalb einer Temperatur (Aₛ; A_{f}), bei der eine Umwandlung vom ersten in den zweiten Zustand auftritt.

3. Verfahren nach Anspruch 1, **dadurch gekennzeichnet, dass** der Schaltschritt die folgenden Schritte umfasst:
- Erhitzen der Anordnung von Stellgliedern (1) von der Umgebungstemperatur für die Benutzung auf eine Temperatur bei oder oberhalb einer Temperatur (Aₛ; A_{f}), bei der eine Umwandlung vom ersten in den zweiten Zustand auftritt, so dass sich die gesamte Anordnung von Stellgliedern (1) entweder ganz oder teilweise im zweiten Zustand befindet,
- Auswählen zumindest eines der Stellglieder (1), das einem vorbestimmten Öffnungsdruck oder Strömungswiderstand entspricht,
- individuelles Abkühlen der ausgewählten Stellglieder auf eine Temperatur bei oder unterhalb einer Temperatur (Mₛ; M_{f}), bei der eine Umwandlung vom zweiten in den ersten Zustand auftritt.

4. Verfahren nach einem der Ansprüche 1 bis 3, **dadurch gekennzeichnet, dass** die benannte Umgebungstemperatur für die Benutzung die Körpertemperatur ist.

5. Verfahren nach Anspruch 4, **dadurch gekennzeichnet, dass** das FGL-Material eine Hysterese zwischen 10 und 40 Grad Celsius besitzt.

6. Verfahren nach einem der Ansprüche 1 bis 5, **dadurch gekennzeichnet, dass** das FGL-Material im ersten und zweiten Zustand die gleiche Gestalt besitzt.

7. Einstellbares Mikroventil mit einem Basiselement (2) mit zumindest einem Durchgang (3) für den Flüssigkeitsstrom, zumindest einer Anordnung von aus einem FGL-Material gefertigten Stellgliedern, die auf einer Aussenseite des Basiselements (2) angeordnet sind, Mitteln (4) zum Abkühlen der Stellglieder und Mitteln (5) zum Erhitzen der Stellglieder, **dadurch gekennzeichnet, dass** das FGL-Material eine Hystereseschleife zwischen einer ersten und einer zweiten Temperatur (Mf, Af) aufweist und so ausgewählt ist, dass eine Umgebungstemperatur für die Benutzung des Ventils in diese Hystereseschleife fällt, so dass das FGL-Material bei dieser Umgebungstemperatur für die Benutzung also einen ersten und einen zweiten stabilen Zustand sowie einen Elastizitätsmodul besitzt, der im ersten Zustand niedriger als im zweiten Zustand ist.

8. Einstellbares. Mikroventil nach Anspruch 7, **dadurch gekennzeichnet, dass** das FGL-Material im ersten und zweiten Zustand die gleiche Gestalt besitzt.

9. Einstellbares Mikroventil nach Anspruch 7 oder 8, **dadurch gekennzeichnet, dass** es weiter ein elastisches Element (7) umfasst, das eine Kugel (6) im Lager einer Öffnung (3) sichert, und **dadurch**, dass die Stellglieder (1) so gestaltet sind, dass ihre jeweiligen freien Enden mit dem elastischen Element (7) wechselwirken, wodurch dessen Elastizität verändert werden kann.

10. Einstellbares Mikroventil nach Anspruch 7 oder 8, **dadurch gekennzeichnet, dass** die Stellglieder (1, 11) so gestaltet sind, dass ihre jeweiligen freien Enden entsprechende Öffnungen (3; 10) im Basiselement (2; 9) verschliessen.

11. Einstellbares Mikroventil nach einem der Ansprüche 7 bis 10, **dadurch gekennzeichnet, dass** die Abkühlmittel aus zumindest einer Peltier-Zelle (4) bestehen, die in der Nähe der Anordnung von FGL-Stellgliedern (1) in das Basiselement (2) integriert ist.

12. Einstellbares Mikroventil nach einem der Ansprüche 7 bis 11, **dadurch gekennzeichnet, dass** die Stellglieder (1) aus TiNi gefertigt sind.

13. Implantierbares Mikroventil mit einem einstellbaren Öffnungsdruck, **dadurch gekennzeichnet, dass** es eine obere Abdeckung (13) mit einem Flüssigkeitsausgang (14) und eine untere Abdeckung (15) mit einem Flüssigkeitseingang (16) besitzt und ein Mikroventil (12) nach einem der Ansprüche 7 bis 12 verkörpert, und **dadurch**, dass es weiter elektronische Mittel (18) und eine Antenne (17) zur Stromversorgung und zur Steuerung der Betätigung des Ventils (12) vermittels Telemetrie über eine externe Einheit umfasst.

14. Implantierbare Pumpe, **dadurch gekennzeichnet, dass** sie eine obere Abdeckung (23) mit einem Flüssigkeitsausgang (24), ein unter Druck stehendes Reservoir (20), eine Ventilgruppe (21) nach Anspruch 7 umfasst und dass es weiter elektronische Mittel sowie eine Antenne zur Stromversorgung und zur Steuerung der Pumpenbetätigung vermittels Telemetrie über eine externe Einheit umfasst.
